# EUROPÄISCHE PATENTSCHRIFT

(11) **EP 2 119 432 B1**
(45) Veröffentlichungstag und Bekanntmachung des Hinweises auf die Patenterteilung: **12.10.2016**
(21) Anmeldenummer: 09010694.9
(22) Anmeldetag: 19.07.2006
(51) Int. Cl.: A61K 8/49, A61K 8/44, A61K 8/42, A61Q 17/02, A01N 47/16, A01N 37/20, A01N 37/18, A61K 8/35, A61K 8/37, A61K 8/31, A61K 8/34, A01N 37/10, A01N 37/02, A01N 43/16, A01N 35/02, A01N 27/00, A01N 31/02, A01N 43/36

(54) **Repellentien mit verstärkter Abwehrwirkung**
Insect repellents with improved activity
Insectifuges avec action fortifiée

(30) Priorität: 20.07.2005 DE 102005033845
(43) Veröffentlichungstag der Anmeldung: 18.11.2009
(62) Teilanmeldung aus: 06117445.4
(73) Patentinhaber: BEIERSDORF AG, 20245 Hamburg (DE)
(72) Erfinder: Hamer, Gunhild, 22455 Hamburg (DE); Kröpke, Rainer, 22869 Schenenfeld (DE); Schulz, Jens, 25462 Rellingen (DE); Sibbers, Ursula, 23863 Bargfeld-Stegen (DE)

(56) Entgegenhaltungen:
- WO-A-98/18891
- WO-A-03/013243
- WO-A1-96/39827
- WO-A2-03/020232
- DE-A1- 10 349 666
- US-A- 5 665 781
- US-B1- 6 660 288
- DATABASE WPI Week 199202 Thomson Scientific, London, GB; AN 1992-013544 XP002408597 & JP 03 264504 A (OGAWA KORYO KK) 25. November 1991 (1991-11-25)

## Beschreibung

Die vorliegende Erfindung betrifft eine kosmetische Zubereitung enthaltend 3-(N-n-Butyl-N-acetyl-amino)propionsäuraethylester.

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenschutzmittel" nicht nur solche Präparate zu verstehen, die gegen Insekten (insbesondere Mücken) wirksam sind. Vielmehr gilt das nachstehend Gesagte selbstverständlich auch für solche Präparate, die andere Blut saugende oder beißende Parasiten und/oder Lästlinge (z.B. Spinnen, Flöhe, Milben) abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitkeln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hoch siedende Flüssigkeiten oder niedrig schmelzende bzw. sublimierende kristalline Stoffe, die bei Raumtemperatur langsam verdampfen. Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur aufweist:

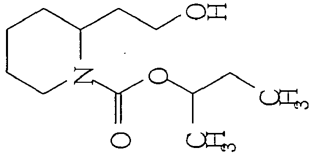

Ein weiterer häufig eingesetzter Repellent-Wirkstoff ist der 3-(N-n-Butyl-N-acetyl-amino)propionsäureethylester (auch als Ethylbutylacetylaminopropionat oder Repellent 3535 bezeichnet), welcher sich durch die folgende Strukturformel auszeichnet:

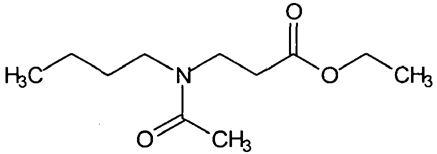

Nicht zuletzt kennt der Fachmann den Repellent-Wirkstoff N,N-Diethyl-3-methylbenzamid (Handelsbezeichnung: Meta-delphene, DEET), der di folgende Struktur aufweist:

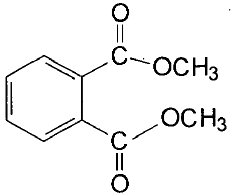

Mücken, Zecken und andere Insekten im Sinne der vorliegenden Offenbarung werden in erster Linie durch eine komplexe Duftmischung aus Milchsäure, Fettsäuren und Aminosäuren angelockt, die von der menschlichen Haut verströmt werden und den Menschen wie eine Duftglocke umgeben. Es ist nun ein Nachteil an Insektenabwehrmitteln des Standes der Technik, dass derartige Zubereitungen die natürliche, menschliche Duftkomposition nicht direkt maskieren, sondern ihr vielmehr eine zusätzliche, auf Insekten abstoßend wirkende Duftnote (die des Repellent) hinzufügen. Um das anziehende und abstoßende Reizpotential auf die Insekten über einen längeren Zeitraum zugunsten der abstoßenden Reizwirkung zu verschieben, ist es nach dem Stande der Technik notwendig, relativ große Mengen an Repellent-Wirkstoffen einzusetzen.

Zubereitungen mit einer hohen Konzentration an Repellentien können jedoch bei Menschen mit besonders empfindlicher Haut in Einzelfällen zu Hautirritationen führen. Ferner haben die Repellent-Wirkstoffe einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Es waren daher die Aufgabe der vorliegenden Erfindung, die Mängel des Standes der Technik zu beseitigen und ein Insektenabwehrmittel zu entwickeln, welches mit einer reduzierten Menge an Repellent-Wirkstoffen zu einer gleichen Wirkung wie herkömmliche Insektenabwehrmittel kommt. Es war eine weitere Aufgabe der vorliegenden Erfindung, ein Insektenabwehrmittel zu entwickeln, welches ein reduziertes Hautreizungspotential aufweist. Nicht zuletzt war des die Aufgabe ein Insektenabwehrmittel zu entwickeln, welches einen für die menschliche Nase angenehm wohlriechenden Duft verströmt.

Überraschend gelöst werden die Aufgaben durch eine kosmetische Emulsion gemäß Anspruch 1.

Dabei ist es erfindungsgemäß bevorzugt, wenn als Verbindungen aus der Gruppe b) Hexylsalicylat und/oder Linaylacetat eingesetzt werden.

Zwar kennt der Stand der Technik die DE 10349666, WO 98/18891, WO 03/0123243, WO 03/020232, US 6660288, WO 96/39827, US 6665781 sowie den Datenbank-Eintrag Database WPI, week 199202, Thomson Scientific, London, GB, AN1992-013544, XP002408597 & JP 3264504, doch konnte dieser Stand der Technik nicht den Weg zur vorliegenden Erfindung weisen.

Es ist erfindungsgemäß vorteilhaft, wenn die Repellentien in einer Gesamtkonzentration von 0,1 bis 40 Gewichts-% und erfindungsgemäß bevorzugt, wenn die Repellentien in einer

Gesamtkonzentration von 10 bis 20 Gewichts-%, jeweils bezogen auf das Gesamtgewicht der Zubereitung, in dieser enthalten sind.

Es ist erfindungsgemäß vorteilhaft, wenn die erfindungsgemäße Zubereitung Hexylsalicylat in einer Konzentration von 1 ppm bis 1,0 Gewichts-% und bevorzugt in einer Konzentration von 0,015 bis 0,065 Gewichts-%, bezogen auf das Gesamtgewicht der Zubereitung, enthält.

Es ist erfindungsgemäß vorteilhaft, wenn die erfindungsgemäße Zubereitung Linaylacetat in einer Konzentration von 1 ppm bis 1,0 Gewichts-% und bevorzugt in einer Konzentration von 0,015 bis 0,065 Gewichts-%, bezogen auf das Gesamtgewicht der Zubereitung, enthält.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, W/S, S/W oder multiple Emulsion, Makro- oder Mikroemulsion), einer Dispersion, einer Pickering-Emulsion vorliegt. Dabei ist es erfindungsgemäß bevorzugt, wenn die kosmetische Zubereitung in Form einer Emulsion vorliegt.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Erfindungsgemäß ist ein Packmittel aus Polyethylen, Polypropylen oder Polyethylenterephthalat enthaltend eine erfindungsgemäße Zubereitung.

Das erfindungsgemäße Packmittel, in dem die erfindungsgemäße Zubereitung aufbewahrt wird, liegt erfindungsgemäß vorteilhaft in Form einer Tube oder einer Kunststoff-Flasche vor. Dabei sind Kunststoff-Flaschen erfindungsgemäß bevorzugt. Eine erfindungsgemäß besonders bevorzugte Ausführungsform stellen Kunststoff-Flaschen mit aufgesetztem Pumpspender dar, mit welchem die erfindungsgemäße Zubereitung aus dem Packmittel gefördert und ggf. versprüht werden kann.

Es ist erfindungsgemäß bevorzugt, wenn die erfindungsgemäße Zubereitung in einem Packmittel aus Polyethylen (PE) aufbewahrt wird. Erfindungsgemäß besonders bevorzugt ist dabei der Einsatz von "high density polyethylene"(HDPE nach DIN 7728, Tl. 1, 01/1988). Erfindungsgemäß ist insbesondere die Verwendung der erfindungsgemäßen Zubereitung zur Prophylaxe von Mückenstichen und Zeckenbissen.

Erfindungsgemäß ist die Verwendung von Verbindungen gewählt aus der Gruppe Hexylsalicylat, Linaylacetat, zur Abwehr von Mücken, Sandfliegen, Läusen, Bremsen, Wespen, Bienen, Fliegen und Zecken.

Erfindungsgemäß ist die Verwendung von Verbindungen gewählt aus der Gruppe Hexylsalicylat, Linaylacetat, zur Herstellung einer kosmetischen Zubereitung zur Abwehr von Mücken, Sandfliegen, Läusen, Bremsen, Wespen, Bienen, Fliegen und Zecken.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

**Spray mit Repellent**

| | **1** | **2** | **3** | **4** | **5** |
|---|---|---|---|---|---|
| Ethylbutylacetylaminopropionat | 5 | 35 | 15 | 20 | 7,5 |
| N, N-Diethyltoluolamid | 5 | --- | --- | 1 | --- |
| Icaridin | 5 | 3,0 | 2,0 | 1,5 | 12,5 |
| 1-(3-Cyclohexen-1-yl-carbonyl)-2-methylpiperidin | 5 | --- | 2 | 1 | --- |
| Hammamelis-Extrakt | 0,2 | --- | --- | 1 | 0,2 |
| Aloe Vera Extrakt | --- | --- | 1 | 1 | 5,0 |
| Tocopherol | 0,5 | 0,5 | 0,75 | 0,25 | 0,1 |
| Meeresextrakt | 0,5 | --- | 0,75 | 3,0 | 0,25 |
| Panthenol | 0,5 | --- | 0,75 | 0,25 | 0,1 |
| Hexylsalicylat | 0,1 | 0,015 | 0,5 | 75 ppm | 0,0065 |
| Parfum | q,s, | q,s, | q,s, | q,s, | q,s, |
| Sorbitol | 10 | --- | --- | 5 | --- |
| Butylenglykol | --- | --- | | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

## Patentansprüche

1. Kosmetische Zubereitung in Form einer Emulsion enthaltend
a) 3-(N-n-Butyl-N-acetyl-amino)propionsäureethylester sowie
b) ein oder mehrere Verbindungen gewählt aus der Gruppe Hexylsalicylat, alpha-Isomethylionon und Hexylcinnamal.

2. Kosmetische Zubereitung nach Anspruch 1, **dadurch gekennzeichnet, dass** die Zubereitung weitere kosmetische Wirk- und Pflegestoffe enthält.

3. Kosmetische Zubereitung nach einem der vorhergehenden Ansprüche, **dadurch gekennzeichnet, dass** als weitere Pflegestoffe Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden

4. Packmittel aus Polyethylen, Polypropylen oder Polyethylenterephthalat enthaltend eine Zubereitung nach einem der vorhergehenden Ansprüche.

5. Tuch oder Pflaster getränkt mit einer Zubereitung nach einem der vorhergehenden Ansprüche.

## Claims

1. Cosmetic preparation in the form of an emulsion comprising
a) 3-(N-n-butyl-N-acetylamino)propionic acid ethyl ester and
b) one or more compounds selected from the group hexyl salicylate, alpha-isomethylionone and hexylcinnamal.

2. Cosmetic preparation according to Claim 1, **characterized in that** the preparation comprises further cosmetic active ingredients and care substances.

3. Cosmetic preparation according to one of the preceding claims, **characterized in that** the further care substances used are niacinamide, panthenol, Aloe vera, hammamelis extract, polidocanol, vitamin E, vitamin E derivatives, vitamin A, vitamin A derivatives, vitamin C, vitamin C derivatives, coenzyme Q10, creatine, taurine, alpha-glycosylrutin in concentrations of from 0.1 to 30% by weight, based on the total weight of the preparation.

4. Packaging made of polyethylene, polypropylene or polyethylene terephthalate comprising a preparation according to one of the preceding claims.

5. Wipe or plaster impregnated with a preparation according to one of the preceding claims.

## Revendications

1. Préparation cosmétique sous forme d'une émulsion, contenant
a) de l'ester éthylique de l'acide 3-(N-n-butyl-N-acétylamino)propionique ainsi que
b) un ou plusieurs composés choisis dans le groupe formé par le salicylate d'hexyle, l'alpha-isométhylionone et l'hexylcinnamal.

2. Préparation cosmétique selon la revendication 1, **caractérisée en ce que** la préparation contient d'autres substances actives et de soin cosmétiques.

3. Préparation selon l'une quelconque des revendications précédentes, **caractérisée en ce qu'**on utilise, comme autres substances de soin, du niacinamide, du panthénol, de l'Aloe vera, de l'extrait d'hamamélis, du polidocanol, de la vitamine E, des dérivés de la vitamine E, de la vitamine A, des dérivés de la vitamine A, de la vitamine C, des dérivés de la vitamine C, de la coenzyme Q10, de la créatine, de la taurine, de l'alpha-glycosylrutine en des concentrations de 0,1 à 30% en poids par rapport au poids total de la préparation.

4. Emballage en polyéthylène, en polypropylène ou en poly(téréphtalate d'éthylène) contenant une préparation selon l'une quelconque des revendications précédentes.

5. Tissu ou emplâtre imbibé d'une préparation selon l'une quelconque des revendications précédentes.
